# EUROPÄISCHE PATENTANMELDUNG

(11) **EP 1 148 063 A1**
(43) Veröffentlichungstag der Anmeldung: **24.10.2001**
(21) Anmeldenummer: 00108430.0
(22) Anmeldetag: 18.04.2000
(51) Int. Cl.: C07K 1/16, C07K 14/745

(54) **Haemostatisch aktives vWF enthaltendes Präparat und Verfahren zu seiner Herstellung**

(71) Anmelder: Octapharma AG, 8853 Lachen (CH)
(72) Erfinder: Josic, Djuro Professor Dr., 1020 Wien (AT); Stadtler, Monika Dipl.-Ing., 2435 Wienerherberg (AT); Gruber, Gerhard Dr., 1210 Wien (AT)
(74) Vertreter: Meyers, Hans-Wilhelm, Dr.Dipl.-Chem.

(57) **Zusammenfassung**

Verfahren zur Herstellung eines haemostatisch aktiven von Willebrand-Faktors (vWF) enthaltenden Präparates aus einer Fraktion humanen Plasmas durch eine chromatographische Reinigung einer vWF enthaltenden Plasmafraktion an einem Anionenaustauschermaterial, das die Anionenaustauschergruppen an gepfropften polymeren Strukturen aufweist (Tentakelmaterialien), Sammeln einer vWF enthaltenden Fraktion, gefolgt von einer Reinigung dieser Fraktion mittels Gelpermeation zur Herstellung eines gereinigten hitzestabilen vWF enthaltenden Präparates und Erhitzen des Präparates zur Inaktivierung von Viren.

## Beschreibung

Gegenstand der vorliegenden Erfindung ist ein Verfahren zur Herstellung eines virusinaktivierten haemostatisch aktiven von Willebrand-Faktors (vWF) enthaltenden Präparates aus einer Fraktion humanen Plasmas sowie ein haemostatisch aktives vWF enthaltendes Präparat erhältlich nach dem erfindungsgemäßen Verfahren.

Die von Willebrand Erkrankung ist eine Blutgerinnungsstörung, die anders als bei der Hämophilie A oder B, als Blutung in weichem Gewebe, insbesondere Schleimhäuten, auftritt. Als Ursache für diese Erkrankung wurde ein Mangel an dem sogenannten von Willebrand Faktor (vWF) erkannt. Dieser Faktor wurde den Patienten bislang durch Faktor VIII Präparate verabreicht, in denen der vWF ebenfalls enthalten ist, allerdings oft in nur unzufriedenstellender Qualität und unbefriedigender Menge. Zwar ist die gentechnische Herstellung des vWF gelungen, aber es besteht noch immer ein großes Bestreben, verbesserte Reinigungsmethoden zur Herstellung des vWF aus Spenderblut bzw -plasma anwenden zu können.

In EP-A-0 934 748 wird ein hoch reiner vWF mit einem Gehalt von mindestens 15 % vWF Multimeren, bestehend aus 13 oder mehr Protomeren (HMW - VWF Multimeren) beschrieben. Dieser wird erhalten durch mehrstufige chromatographische Reinigung, wobei initial eine Gelfiltration "group separation mode" anstelle der Kryopräzipation vorgenommen werden kann. Zur Inaktivierung von Viren wird beispielsweise eine Behandlung mit Lösungsmittel und Detergenz sowie eine Hitzebehandlung im lyophilisierten Zustand für 72 h bei 80 °C beschrieben.

Josic et al "Purification of factor VIII and vWF factor from human plasma by anionexchange chromatography", Journal of Chromatography B, 662 (1994)181 - 190; sowie Schwinn et al "A Solvent 1 detergent Treated, Pasteurised and Highly Purified Factor VIII Concentrate", Arzneimittel-Forschung 1 Drug Research 44 (1), 2,188 - 191 (1994) betreffen eine Reinigung von Faktor VIII - vWF Komplex. Dabei wird eine Reinigung über einen lonenaustauscher und es werden zwei unabhängige Schritten zur Inaktivierung von Viren durchgeführt. Es erfolgt eine Behandlung mit Solvens/Detergenz und eine Pasteurisierung (10 h bei 63 °C) in Gegenwart von Stabilisatoren. Obwohl der Faktor VIII durch die Pasteurisierung kaum denaturiert wird (95% Restaktivität), wird der vWF in seiner Aktivität und Multimerenverteilung verändert (85% Restantigen) und Verlust von Hochmolekularen Multimeren. Daraus resultiert ein vWF Antigen zu Aktivität Verhältnis von 2,2 (Aktivität zu Antigen = 0,5).

Die US-A-5 714 590 beschreibt eine Reinigung eine Faktor VIII Komplexes durch Ionenaustauschchromatographie. Dazu wird ein EMD - TMAE - Fractogel Säulenmaterial eingesetzt, welches auch für die Gelpermeation verwendet wird. Zur Virusinaktivierung wird die Behandlung mit Solvens/Detergenz vorgeschlagen. Eine Hitzebehandlung ist nicht beschrieben. Das erhaltene Produkt enthält auch den vWF, wobei nur die vWF Antigenizität angegeben wird.

Das der Erfindung zu Grunde liegende Problem besteht in der Schaffung eines neuen Verfahrens zur Reingung des vWF aus humanem Plasma. Das Verfahren soll dabei den vWF in verbesserter Qualität und Wirksamkeit liefern.

Das der Erfindung zugrunde liegende technische Problem wird gelöst durch ein Verfahren zur Herstellung eines haemostatisch aktiven von Willebrand-Faktors (vWF) enthaltenden Präparates aus einer Fraktion humanen Plasmas, welches sich dadurch auszeichnet, dass zunächst eine chromatographische Reinigung einer vWF enthaltenden Plasmafraktion an einem Anionenaustauschermaterial, das die Anionenaustauschergruppen an gepfropften polymeren Strukturen aufweist (Tentakelmaterialien), durchgeführt wird, Sammeln einer vWF enthaltenden Fraktion gefolgt von einer Reinigen der Fraktion mittels Gelpermeation und erhitzen des Präparats zur Inaktivierung von Viren. Die im erfindungsgemäßen Verfahren einsetzbaren Tentakelmaterialien werden insbesondere in der EP-A-0 337 144 offenbart. Auf diese Publikation wird ausdrücklich Bezug genommen. Es handelt sich dabei vorzugsweise um an Diole des Fractogel® gepfropfte Polyacrylamidtentakel-Polymere der Firma Merck, Darmstadt, Deutschland.

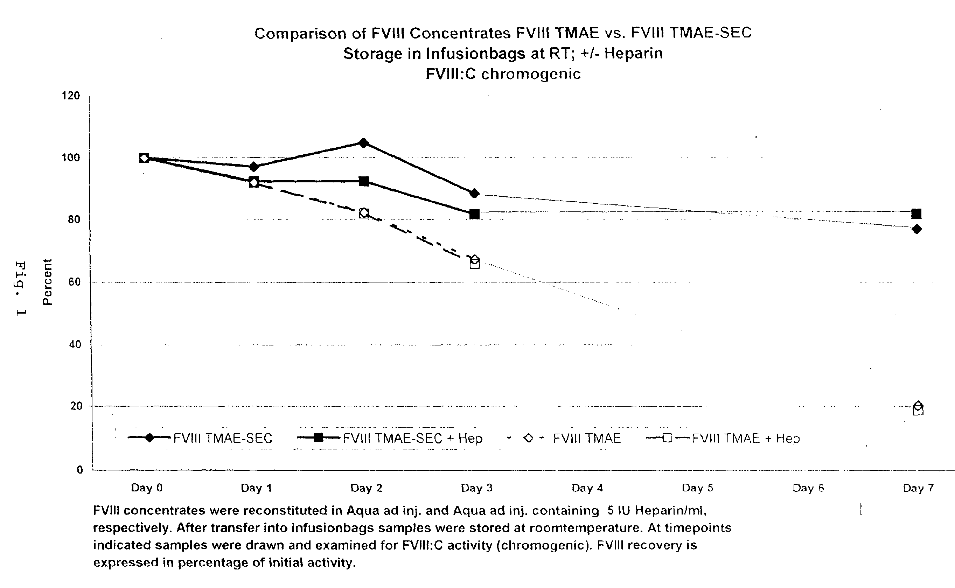

Die Fig. 1 zeigt die Stabilität des erfindungsgemäß erhältlichen Produkts gegenüber den nur durch Anionenaustauscherchromatographie gereinigten Produkten.

Diese Verfahrensweise führt überraschenderweise zu einem Produkt, mit einem hohen vWF-Gehalt im Verhältnis zu F VIII. Die erfindungsgemäße Abfolge führt durch den Gelpermeationsschritt zu einer Entfernung von Inter-α-Trypsininhibitor sowie anderer Faktoren, die in bekannten Herstellungsverfahren zu Verminderung der Qualität des Produktes beigetragen haben können. Durch die erfindungsgemäße Abfolge der chromatographischen Schritte wird ein vWF Präparat erhalten, welches überraschend stabil ist. Die mittels des erfindungsgemäßen Verfahrens erhältliche vWF Fraktion ist überraschenderweise in einer wässrigen Lösung bei Raumtemperatur über längere Zeit (gemessen wurde bis zu einer Woche) im wesentlichen stabil. Das erfindungsgemäße Präparat enthält den vWF darüber hinaus in nativer Form und frei von Fragmenten.

Vorzugsweise zeigt das hitzestabile Präparat sowie das erhitzte Präparat nur einen Peak in der HPLC Gelfiltrationsanalyse .

Insbesondere kann das hitzestabile Präparat gemäß des Verfahrens in lyophilisiertem Zustand bei einer Temperatur von mehr als 80 °C erhitzt werden. In einer weiteren Ausführungsform des erfindungsgemäßen Verfahrens wird das Präparat bei einer Temperatur von mehr als 90 °C, insbesondere bei 1 00 °C erhitzt. Erfindungsgemäß erfolgt die Erhitzung dabei vorzugsweise für einen Zeitraum von mindestens 1 h bis zu 5 h, insbesondere 2 bis 3 h. Damit werden alle transfusionsrelevanten Viren, insbesondere nicht lipidumhüllte Viren, wie Parvo-Viren oder Hepatitis-A-Viren, inaktiviert. Erstaunlicherweise das erfindungsgemäß erhältliche Produkt diese anspruchvolle Hitzebehandlung ohne nennenswerten Verlust an Aktivität aus.

Erfindungsgemäß kann eine Reinigung der den vWF enthaltende Fraktion zusätzlich durch mindestens eine adsorptions- und/oder affinitätschromatographische Behandlung erfolgen.

Erfindungsgemäß läßt sich ebenfalls eine vWF enthaltende Fraktion verwenden, die aus gepooltem humanen Plasma erhalten wird und mittels des erfindungsgemäßen Verfahren gereinigt werden kann.

Gegenstand der vorliegenden Erfindung ist ebenfalls ein haemostatisch aktives vWF enthaltendes Präparat erhältlich nach dem erfindungsgemäßen Verfahren.

Das erfindungsgemäße Präparat ist frei von infektiösen von Blut übertragbaren Viren, insbesondere HIV, HBV, HCV, HAV und Parvo Viren.

Insbesondere enthält das erfindungsgemäße Präparat den vWF in hochmolekularer Form, welches nach der elektrophoretischen Analyse mindestens 13 von 16 Multimerenbanden zeigt. Die Anzahl der Multimeren und deren Verteilungsmuster ist ein Maß für die Ähnlichkeit einer vWF-Präparation mit dem nativen vWf im Plasma. Weiterhin beträgt das Verhältnis von Ristocitin-Cofaktor als Surrogatmarker für die vWF Aktivität zu vWF Antigen beim erfindungsgemäßen Präparat mindestens 0,8. Dies ist ein weiteres Maß für die Integrität des vWF-Moleküls.

Das erfindungsgemäße Präparat enthält vorteilhafterweise weiterhin den Faktor VIII in nativer Form. Die Verwendung von Komplexen aus F VIII und vWF zur Behandlung der von Willebrand Erkrankung hat sich in der Klinik besonders bewährt.

Des Weiteren wurden dem erfindungsgemäßen Präparat die folgenden Proteine Immunglobuline, Fibrinogen, Fibronektin sowie Inter α-Trypsininhibitor bis unter eine immunchemische Nachweisgrenze entfernt. Darauf beruht vermutlich die außerordentliche Stabilität des erfindungsgemäßen Produkts.

Die Erfindung wird anhand der folgenden Beispiele näher erläutert.

### Beispiele

Etwa 47 kg Kryopräzipitat werden mit 15 % (w/w) einer 2 %igen (w/w) Aluminiumhydroxidlösung behandelt. Nach Zentrifugation wird die Probe mit 0.3 % TnBP/ 1 % Triton X 100 virusinaktiviert. Nach Ölextraktion schließt sich eine Anionenaustauscher-Chromatographie an Fractogel EMD TMAE (Tentakelgel der Fa Merck, Darmstadt, Deutschland) an.

Die Pufferbedingungen sind wie folgt:
Puffer AM, erster Waschschritt:
   120 mM NaCl, 10 mM Na-citrat, 120 mM Glycin, 1 mM CaCl₂ pH 6.9 - 7.1; Osmolalität: 360-400 mosmol/kg.
Puffer BM, zweiter Waschschritt:
   235 mM NaCl, 10 mM Na-citrat, 120 mM Glycin, 1 mM CaCl₂ pH 6.9 - 7.1; Osmolalität: 570 - 610 mosmol/kg.
Puffer CM, Elution:
   700 mM NaCl, 10 mM Na-citrat, 120 mM Glycin, 1 mM CaCl₂ pH 6.9-7.1; Osmolalität: 1380 - 1480 mosmol/kg ;
Puffer DM, dritter Waschschritt:
   1.5 M NaCl

Die Fraktionen mit vWF/FVIII Aktivität werden einer GrößenausschlussChromatographie an einem Fractogel EMD Bio SEC (Gel der Fa. Merck, Darmstadt, Deutschland) unterzogen. Nach einer Ultra/Diafiltration erfolgt die Formulierung mit folgender Zusammensetzung: 400 mM NaCl, 10 mM Nacitrat, 1 mM CaCl₂, 133 mM Glycin, 1% Saccharose.

Daran schließt sich eine Sterilfiltration an. Das Präparat wird abgefüllt und lyophilisiert. Es schließt sich eine Wärmebehandlung bei einer Temperatur von 100 °C an. Die Wärmebehandlung dauert 120 Minuten.

Die Fig. 1 zeigt die Stabilität des erfindungsgemäß erhältlichen Produkts gegenüber den nur durch Anionenaustauscherchromatographie gereinigten Produkten. Das erfindungsgemäße Produkt wurde nach der Anionenaustauscherchromatographie mit einer Größenausschlusschromatographie behandelt. Es zeigt sich, dass bei Lagerung mit oder ohne Heparin in wässrigar Lösung bei Raumtemperatur, das erfindungsgemäße Produkt noch eine Aktivität von 80 % aufwies, während Produkte, die keine Größenausschlusschromatographie erfahren hatten, nach 7 Tagen nur noch eine Aktivität von 20 % aufwiesen.

## Patentansprüche

1. Verfahren zur Herstellung eines haemostatisch aktiven von Willebrand-Faktors (vWF) enthaltenden Präparates aus einer Fraktion humanen Plasmas durch
I. eine chromatographische Reinigung einer vWF enthaltenden Plasmafraktion an einem Anionenaustauschermaterial, das die Anionenaustauschergruppen an gepfropften polymeren Strukturen aufweist (Tentakelmaterialien), Sammeln einer vWF enthaltenden Fraktion, gefolgt von einer
II. Reinigung dieser Fraktion mittels Gelpermeation zur Herstellung eines gereinigten hitzestabilen vWF enthaltenden Präparates und
III. Erhitzen des Präparates zur Inaktivierung von Viren.

2. Verfahren nach Anspruch 1, **dadurch gekennzeichnet, dass** das hitzestabile Präparat sowie das erhitzte Präparat nur einen Peak in der HPLC Gelfiltrationsanalyse zeigt.

3. Verfahren nach Anspruch 1 oder 2, **dadurch gekennzeichnet, dass** das hitzestabile Präparat in lyophilisiertem Zustand bei einer Temperatur von mehr als 80 °C erhitzt wird.

4. Verfahren nach Anspruch 3, **dadurch gekennzeichnet, dass** das Präparat bei einer Temperatur von mehr als 90 °C, insbesondere bei 1 00 °C er-hitzt wird.

5. Verfahren nach Anspruch 3 oder 4, **dadurch gekennzeichnet, dass** das Präparat mindestens 1 h bis zu 5 h, vorzugsweise 2-3 h lang erhitzt wird.

6. Verfahren nach einem der Ansprüche 1-5, **dadurch gekennzeichnet, dass** die Fraktion zusätzlich durch mindestens eine Adsorptions- und/oder Affinitätschromatographie gereinigt wird.

7. Verfahren nach einem der Ansprüche 1-6, **dadurch gekennzeichnet, dass** das Reinigen mittels Gelpermeation als abschließende Maßnahme zur Proteinreinigung durchgeführt wird.

8. Verfahren nach einem der Ansprüche 1-7, **dadurch gekennzeichnet, dass** die Fraktion aus gepooltem humanen Plasma gereinigt wird.

9. Haemostatisch aktives vWF enthaltendes Präparat erhältlich nach einem der Ansprüche 1-8.

10. Präparat nach Anspruch 9, **dadurch gekennzeichnet, dass** das Präparat keine infektiösen von Blut übertragbaren Viren, insbesondere HIV, HBV, HCV, HAV und Parvo Viren, enthält.

11. Präparat nach Anspruch 9 oder 10, **dadurch gekennzeichnet, dass** es den vWF in hochmolekularer enthält und nach der elektrophoretischen Analyse mindestens 13 von 16 Multimerenbanden zeigt.

12. Präparat nach einem der Ansprüche 9-11, **dadurch gekennzeichnet, dass** das Verhältnis von vWF Aktivität gemessen als Ristocitin Cofaktor zu vWF Antigen mindestens 0,8 beträgt.

13. Präparat nach einem der Ansprüche 9-12, **dadurch gekennzeichnet, dass** es weiterhin den Faktor VIII in nativer Form enthält.

14. Präparat nach einem der Ansprüche 9-13, **dadurch gekennzeichnet, dass** es Immunglobuline, Fibrinogen, Fibronektin sowie Inter α-Trypsininhibitor unter der immunchemischen Nachweisgrenze enthält.

15. Präparat nach einem der Ansprüche 9-14, **dadurch gekennzeichnet, dass** es den vWF in nativer Form und frei von Fragmenten enthält.
